# EUROPEAN PATENT SPECIFICATION

(11) **EP 2 427 566 B1**
(45) Date of publication and mention of the grant of the patent: **28.10.2015**
(21) Application number: 10718580.3
(22) Date of filing: 05.05.2010
(51) Int. Cl.: A23L 1/03, C12P 7/52, C12R 1/25

(54) **PROBIOTIC MICROORGANISMS ISOLATED FROM DONKEY MILK**
AUS ESELSMILCH ISOLIERTE PROBIOTISCHE MIKROORGANISMEN
MICRO-ORGANISMES PROBIOTIQUES ISOLÉS DU LAIT D'ÂNESSE

(30) Priority: 05.05.2009 EP 09159379
(43) Date of publication of application: 14.03.2012
(73) Proprietor: Eurolactis Group S.A., 1420 Luxembourg (LU)
(72) Inventor: NAZZARO, Filomena, 80055 Portici (NA) (IT); ORLANDO, Pierangelo, 80078 Pozzuoli (NA) (IT); CONTI, Amedeo, 14022 Castelnuovo don Bosco (AT) (IT)
(74) Representative: GLN SA
(86) International application number: PCT/EP2010/056117
(87) International publication number: WO 2010/128084

(56) References cited:
- C CHIAVARI, F COLORETTI, M NANNI, E SORRENTINO, L GRAZIA: "Use of donkey^s milk for a fermented beverage with lactobacilli" LAIT, vol. 85, 1 October 2005 (2005-10-01), pages 481-490, XP002546786
- LARA-VILLOSLADA FEDERICO ET AL: "Beneficial effects of probiotic bacteria isolated from breast milk." THE BRITISH JOURNAL OF NUTRITION OCT 2007, vol. 98 Suppl 1, October 2007 (2007-10), pages S96-100, XP002546804 ISSN: 0007-1145

## Description

### Technical Field

The invention relates to the field of micro-organisms known as probiotics and their use in the food industries, in human or animal food. In particular, the invention relates to probiotics isolated particularly from raw Donkey milk.

### Background of the invention

The probiotic micro-organisms, or simply probiotics can be defined as micro-organisms which, when administered in adequate doses, are likely to confer a benefit in terms of health or nutrition. They belong to the category of so-called functional foods.

Probiotics are known in dairy products, such as products marketed under the Actimel® or Activia® brands by DANONE, Yakult® brand by Yakult Honsha, or under the LC1® brand by NESTLE. These bacteria belong to different genera and species of lactic acid bacteria, for example Bifidus spp. Lactobacillus casei, Lactobacillus rhamnosus or Lactobacillus johnsonii.

It is generally accepted that probiotics provide a health or nutritional benefit through the influence they can have on the balance of the intestinal flora, though the precise mechanisms by which probiotics act are not always known.

In recent years, the interest in Donkey milk has been considerably increased, mainly due to its composition, so that it may be considered a valid alternative for infant nutrition to powdered milks, soybean milk or other formulas. In fact Donkey milk is viewed as very close to the human milk due to its composition in poly-unsatured fatty acids, its calcium/phosphorous ratio and its protein content. In addition, Donkey milk is rich of lysozyme, a glycosidase capable to hydrolyze the polysaccharides of the microbial cell wall. Several scientific evidence proves the nutritional and health importance of lysozyme. The high content of lactose has a positive effect on the intestinal absorption of calcium and it is responsible for palatability. Even after the first months of life, Donkey milk can enhance bone mineralization and constitutes an important nutritional support in those children with severe Ig-E mediated cow milk protein allergy, thus playing a role in the formation of an efficient immune system. In the elderly age, Donkey milk can exert positive effects against different cardiovascular pathologies and in hypocholesterolemic diets.

Moreover the publication of C. Chiavari et al. " Use of donkey' s milk for a fermented beverage with lactobacilli" , LAIT, vol. 85, 1 October 2005 (2005-10-01), pages 481-490, discloses some lactic acid bacteria isolated from donkey milk.

### Summary of the invention

It is an object of the invention to provide novel probiotic lactic bacteria.

To this end, an embodiment of the invention proposes probiotic lactic bacteria, isolated especially from Donkey milk and selected from the group consisting of strains deposited under the terms of the Budapest Treaty at the "Deutsche Sammlung von Mikroorganismen und Zellkulturen GmbH" (DSMZ, Braunschweig, Germany) on December 8, 2008 under accession number DSM 22098, DSM 22099, DSM 22100, DSM 22102, DSM 22101, and on April 26, 2010 under accession number DSM 23558, DSM 23559 and DSM 23560. The strains deposited under DSM 22102, DSM 22101, DSM 23558, DSM 23559, and DSM 23560 define a new species called hereinafter Lactobacillus asini.

Another embodiment of the invention proposes a composition comprising one or a plurality of the probiotic lactic bacteria species or strains of the invention as mentioned above. Said composition could be a food composition or a beverage composition, for human or animal feed.

A further embodiment of the invention provides a process for manufacturing a food composition or a beverage composition. Said process may comprise at least the steps of:
- inoculating a food product with viable probiotic lactic bacteria of the invention as described above,
- placing the inoculated food product under conditions favorable to the metabolism of said probiotic lactic bacteria
- fermenting said food product until a population of at least [10⁶] CFU / mL of food product is reached.

In another embodiment, the process for manufacturing a food composition or a beverage composition contains at least the steps of:
- fermenting probiotic lactic bacteria as described above until a population of at least [10⁶] CFU / mL is reached,
- protecting said probiotic lactic bacteria, for example by enveloping,
- admixing a food product with said protected probiotic lactic bacteria.

The fermentation is stopped when it has reached the stationary phase of fermentation, preferably within about 24 hours of having placed said probiotic lactic bacteria under conditions favorable to their metabolism.

Another embodiment of the invention proposes the use of probiotic lactic bacteria in the preparation of a composition for treating a disorder associated with the colonization of the mucous membranes by pathogenic microorganisms. Mucous membranes include, but are not limited to the gut mucosa, the stomach mucosa. For instance, pathogenic microorganisms may be enteropathogens. For instance, inhibitory activity of Lactobacillus strains according to the invention, could be against: enteropathogens (for example Salmonella enteriditis, Vibrio cholereae, Escherichia coli).

Another embodiment of the invention proposes the use of probiotic lactic bacteria for protecting fermented food products against food pathogens, such as Lysteria or Salmonella, Campylobacter or Clostridium, by inhibiting the development of such food pathogens.

Another embodiment of the invention proposes the use of probiotic lactic bacteria of the invention as defined above for producing butyric acid during fermentation making use of such bacteria.

Another embodiment of the invention provides a method for producing butyric acid comprising the steps of fermenting probiotic lactic bacteria of the invention under suitable conditions and collecting the butyric acid produced during fermentation by said bacteria.

These and other aspects, features and advantages of the invention will become apparent to those skilled in the art upon reading the disclosure provided here in connection with the attached drawings. The detailed description, while indicating preferred embodiments of the invention, is only given by way of illustration.

### Brief description of the drawings

Figure 1 shows the evolution of the pH during 24h of incubation of clones of the invention and reference strains in donkey milk.
Figure 2 shows a DNA-fingerprinting of 8 clones isolated from raw Donkey milk.
Figures 3a to 3e show the electropherograms of clones 37, 38, 41, 43 and 48, respectively, after DNA-fingerprinting analysis.
Figure 4 shows the result of DNA-DNA hybridization between clones 37, 38 and 48, using L. plantarum DNA labelled by dUTP-digoxigenin as a probe.
Figure 5 shows the result of DNA-DNA hybridization between clones 37, 38 and 48 were DNA-DNA hybridised within L. plantarum, L. paraplantarum and L. pentosus strain-type DNAs, to a probe of labelled DNA from clone 37.

### Detailed description

During the past decades, the role of probiotics on the human health gained great relevance both at scientific and industrial level, by causing a sensible increase in their market request, as well as to a greater production and consumption of products. For the viability aspect it is of prime importance that the probiotic strains have the ability to overcome the extremely low pH of gastric acid and the detergent effect of bile salts and to arrive in a viable physiological state at the site of action: the intestinal epithelium. Probiotic bacteria are capable to colonize the colon, to positively affect the infections outcome by pathogenic bacteria, to stimulate the immune system and to decrease unfavourable metabolites concentration. In addition, some strains, the bacteria of the invention being included, can provide a certain reduction of cholesterol and triacylglycerol plasma concentrations.

During passage in the gastro-intestinal (GI) tract, probiotic cultures are required to tolerate the presence of pepsin and the low pH of the stomach, the protease-rich conditions of the duodenum, and the antimicrobial activity of bile salts. Although the pH of the stomach may increase up to 6.0 or higher after food intake, it generally ranges from 2.5 to 3.5. Following stomach passage, the small intestine is a second major barrier in the GI tract. Although the pH of the small intestine (i.e., 7.0 to 8.5) is more favourable toward bacterial survival, the presence of bile salts may have adverse effects. Traditionally, the ability of a probiotic candidate to survive GI transit is assessed using conventional plating techniques that provide information on the number of viable and reproductive cells during incubation in simulated GI juices as described for instance in an article by Charteris et al. « Development of an in vitro methodology to determine the transit tolerance of potentially probiotic Lactobacillus and Bifidobacterium species in the upper gastrointestinal tract » J. Appl. Microbiol. 84 :759-768 (1998).

Therefore, various cultures based methods, such as resistance to gastric juices, resistance to bile salts, bile salt hydrolyzing capability, hydrophobicity evaluation, antimicrobial activity, antibiotic sensitivity, may be used to characterize probiotic micro-organisms. These tests are fully described in the Examples section below.

In an embodiment of the invention, the probiotic bacteria meet at least one of the following criteria :
- at least 75% CFU/mL are retained after the resistance to gastric juice evaluation as described herein,
- at least 75% CFU/mL are retained after the resistance to gastric juice and bile salts evaluation as described herein,
- a microbial aggregation visible onto a microscopy glass with 0.5 M ammonium sulphate or less, preferably with 0.1 M ammonium sulphate or less, in the hydrophobicity test as described herein,
- a low antimicrobial activity against other Lactobacillus spp, as measured by the halos test, i.e. a halo smaller than 0.5 cm, preferably smaller than 0.2 cm as described herein,
- an inhibitory activity against Pseudomonas aeruginosa, Bacillus cereus, or Escherichia coli, as measured by the halo method, i.e. a halo greater than 0.5 cm, preferably greater than 0.8 cm as described herein.

Clones 37, 38, 48, 43, 41, 32, 34 and 57 represent specific embodiments of the invention. They have been deposited on December 8, 2008 (for Clones 37, 38, 48, 43, 41) and on April 26, 2010 (for Clones 32, 34, 57) with the "Deutsche Sammlung von Mikroorganismen und Zellkulturen GmbH" (DSMZ) in conformity with the Budapest Treaty, under the accession number:

| | |
|---|---|
| Clone 37 | DSM 22098 |
| Clone 38 | DSM 22099 |
| Clone 48 | DSM 22100 |
| Clone 43 | DSM 22102 |
| Clone 41 | DSM 22101 |
| Clone 32 | DSM 23558 |
| Clone 34 | DSM 23559 |
| Clone 57 | DSM 23560. |

Clones 37, 38 and 48 belong to the subspecies Lactobacillus plantarum asini and clones 43, 32, 34, 57 and 41 belong to the species Lactobacillus asini. More particularly, clone 32 belongs to a subspecies called Lactobacillus asini ssp. butyricus, clone 34 belongs to a subspecies called Lactobacillus asini ssp. lactis, and clone 57 belongs to a subspecies called Lactobacillus asini ssp. caudatus.

Said probiotic bacteria may be provided as a fresh culture or as dried food supplement, such as dietary supplement. In the latter case, the biomass may be freeze-dried or sprayed-dried, to provide a high quality culture powder, comprising for example at least 10⁸ CFU/g, preferably over 10⁹ CFU/g.

According to the invention, probiotic bacteria may be used in the preparation of food products or beverages, for human or animal consumption. Food products and beverages include fermented food products, such as fresh dairy products, fermented milks, yoghurts. Milk usually refers to cow milk. In the context of the invention, Donkey milk may be preferred, although other milks may also be used for preparing dairy products, including mare milk, goat milk, camel milk, ewe milk.

Methods for preparing such fermented dairy food products are within the reach of person of ordinary skill in the art. Preferably, the probiotic bacteria a provided or kept in a viable form up until consumption. The milk may be used fresh, or in powder and reconstituted with water. Various ingredients may be added to the milk, to improve fermentation, or to provide additional health benefits to the consumer (such as adding vitamins or minerals).

After fermentation, the milk can be transformed into cottage-cheese or quark, by adding rennet to the fermented milk for instance. Such procedures are well-known to the skilled person.

In another embodiment, the probiotic bacteria are used as an ingredient in a food or beverage composition, without fermentation of said composition. In other words, said probiotic bacteria is a dietary or nutritional supplement. In this case, it is preferred that the probiotic bacteria be provided in a viable form.

Probiotic lactic bacteria according to the invention may also be used to protect food products, such as fermented food products, against food pathogens, such as Lysteria or Salmonella, Campylobacter or Clostridium, by inhibiting the development of such food pathogens. In that case, a composition comprising the desired probiotic bacteria, as disclosed herein, is admixed to a food or beverage product. The latter product may be fermented independently of the composition comprising the desired probiotic bacteria. In any case, it is preferred that fermentation lasts not more than about 24 hours. Usually, the probiotic strains have reached the stationary phase of fermentation within this time period.

In an embodiment of the invention, fermentation is stopped when it has reached the stationary phase of fermentation, preferably within about 24 hours of having placed the inoculated food product under conditions favorable to the metabolism of said probiotic lactic bacteria.

The invention also relates to the use of probiotic lactic bacteria in the preparation of a composition for treating a disorder associated with the colonization of the mucous membranes by pathogenic microorganisms. Mucous membranes include, but are not limited to the gut mucosa and the stomach mucosa. For instance, pathogenic microorganisms may be enteropathogens. For instance, inhibitory activity of Lactobacillus strains according to the invention, could be against: enteropathogens (for example Salmonella enteriditis, Vibrio cholereae, Escherichia coli). This is associated with the probiotic bacteria' s capacity to adhere to epithelium cells, such as gut cells and, as it is currently understood, to exclude to a certain degree, such pathogenic bacteria from the gut. In turn, this could be correlated with the hydrophobicity of the probiotic bacteria.

The invention also relates to the use of probiotic lactic bacteria of the invention for producing butyric acid during fermentation making use of such bacteria, for example the fermentation of food in which such bacteria have been inoculated, and then ingested by the user, the internal fermentation in the colon or the fermentation of an appropriate medium in suitable conditions, allowing to collect the butyric acid produced during fermentation by said bacteria. Preferred bacteria are DSM 23558, DSM 22098, DSM 22100, and DSM 22099.

### EXAMPLES

Culture based methods (probiotic tests, bile salt hydrolyzing capability, hydrophobicity evaluation, antimicrobial activity, antibiotic sensitivity, production of organic acids, evolution of the pH during incubation)

Raw Donkey milk obtained from an organic breeding was serially diluted in sterile physiological solution (NaCl 0.85%) and inoculated onto MRS (Man, de Rogosa and Sharpe) agar plates specific for the isolation of Lactobacillus genus. Plates were incubated in anaerobic conditions (Anaerogen, Oxoid) for 48 h at different temperatures. Lactic acid bacteria isolates (about 150) were randomly selected from MRS-agar plates of the highest dilutions. The isolates were subcultured in MRS-broth and streaked onto MRS-agar.

### Probioticity tests

### 1) Resistance to gastric juices

After growth, each colony was centrifuged, washed in sterile physiological solution, and re-suspended to the original volume with the same solution. The evaluation of gastric juice resistance was assayed according to De Giulio et al. « Use of alginate and cryo-protective sugars to improve the viability of lactic acid bacteria after freezing and freeze-drying » World Journal of Microbiology & Biotechnology 21 :739-746(2005), with some modifications. Aliquots were incubated in a simulated of gastric juice (pepsin 3 g/L, pH 2.5), withdrawn at different time of incubation, until 180 min, and plated. As a negative control, untreated cells were used and inoculated. The resistance to gastric juice was evaluated by the Colony Forming Units (CFU)/mL and compared with the negative control.

### 2) Resistance to gastric and bile salts

The colonies that were resistant to the gastric juices were incubated for a maximum of 3 h in a simulated of bile juice, composed of MRS containing bile salts 0.3%, then they were inoculated onto MRS agar plates, according to De Giulio et al. (2005). The untreated colonies were used as a negative control. The resistance to bile salts was evaluated by the Colony Forming Units (CFU)/mL and compared with the negative control.

### RESULTS

Among 150 bacterial colonies isolated from raw Donkey milk, only 8 were identified as belonging Lactobacillus genus (named provisionally as Clone 32, Clone 34, Clone 37, Clone 38, Clone 41, Clone 43, Clone 48, and Clone 57), exhibited resistance to gastric juice and bile salts and showed about 75% of CFU/mL formed in comparison to the untreated control Lactobacillus put as 100%. Their optimum growth temperature was about 30 to 31°C.

Only 8 of the 150 colonies exhibited a good resistance to the simulated of gastric juice and to bile salts presence. These colonies were then studied for the following activities : hydrophobicity test, screening of cultures for bile salts hydrolyzing activity, antimicrobial activity and antibiotic sensitivity.

### 3) Hydrophobicity test

The capability of colonies to potentially adhere to the intestinal epithelium was evaluated by using the indirect method of hydrophobicity of Strus et al. « The in vitro activity of vaginal Lactobacillus with probiotic properties against Candida » Infectious Diseases in obstetrics and Gynaecology, 13(2) :69-75 (2005) and Ljungh et al. « High surface hydrophobicity of autoaggregating Staphylococcus aureus strains isolated from human infections studied with the salt aggregation test » Infection and immunity, 47: 522-526 (1985). Colonies were grown in MRS broth for 24h. Microbial suspensions were mixed with equal volumes of sulphate ammonium, previously prepared with different molarities (ranging from 20 mM to 4 M). The smallest concentration of sulphate ammonium capable to cause the microbial aggregation visible onto a microscopy glass was inversely related to the salt aggregation test. An isotonic solution was used as a control.

### RESULTS

- visible aggregation with 0.1 M ammonium sulphate: clones 32, 37,43 and 57
- visible aggregation with 0.5 M ammonium sulphate: clones 34,38,41 and 48.

From these data, a stronger in vitro adhesion capability to intestinal epithelium may be hypothesised for clones 32, 37, 43 and 57.

### 4) Screening of cultures for bile salts hydrolyzing activity

Bile salt hydrolysis is an important metabolic reaction in the bile salt metabolism of mammals. In recent years interest has increased to use bile salt hydrolysis to influence the cholesterol metabolism of humans and animals. The hydrolyzing of bile salts and the incorporation of cholesterol into the cellular membrane have the potential to lower serum cholesterol concentrations in humans. The release of free bile salts through the hydrolyzing of conjugated bile salts in the small intestine results in the excretion of more bile salts in the faeces. The primary means by which cholesterol is removed from the body is by excretion in the form of hydrolyzed bile salts. Most conjugated bile salts are re-circulated through the enter hepatic circulation. The bile salts that are excreted must be replaced by new bile acids, which are formed from cholesterol in the body. Thus, the more bile salts that are excreted, the more cholesterol is utilized from the pool within the body. Furthermore, free bile salts do not support the absorption of cholesterol and other lipids from the small intestine as well as do conjugated bile salts.

The bile salts hydrolyzing activity of the colonies was qualitatively evaluated following the method described by Minelli et al. « Assessment of novel probiotic Lactobacillus casei strains for the production of functional dairy foods » Int. Dairy J., 14 : 723-726 (2004). Briefly, overnight liquid cultures of strains (5 µL) were spotted onto MRS agar containing 0.5% conjugated bile salt mixture and 0.37 g/L of CaCl₂, and incubated as above described. The presence of the precipitated bile acid around spots (opaque halo) was considered a positive result, showing the capability of strains to hydrolyze bile salts.

### RESULTS

The capability to hydrolyze bile extracts were qualitatively evaluated. All strains were capable to hydrolyze bile extracts.

### 5) Antimicrobial activity

The inhibition halos test on agar plate assay was employed to investigate the antimicrobial activity of the microbial strains. Samples were tested against the following bacteria :
- non-pathogenic strains: Lactobacillus acidophilus DSM 20079, Lactobacillus casei ATCC 9595, Lactobacillus bulgaricus DSM 20081, Lactobacillus sakei DSM 20494, Lactobacillus rhamnosus DSM 20711 ;
- pathogenic Gram positive strains : Bacillus cereus GN 101, DSM 4313 and DSM 4384, and Enterococcus faecalis ATCC 29212,
- pathogenic Gram negative strains : Escherichia coli (DSM 8579) and Pseudomonas aeruginosa (DSM 50071).

All strains were purchased from Deutsche Sammlung von Mikroorganismen und Zellkulturen GmbH (DSMZ Germany). Each strain was incubated at 37°C for 18 h into its specific growth medium: lactic acid bacteria were grown in Man de Rogosa Sharpe (MRS) broth (Oxoid), E. coli, E. faecalis, P. aeruginosa and B. cereus in Nutrient Broth (Oxoid). The microbial suspensions (1 x 10⁸ CFU/mL) were uniformly spread onto the specific solid media plates. 50 µL of each culture were individually placed on the inoculated plates. After 30 min standing under sterile conditions at room temperature, plates were incubated at 37°C for 24 to 48 h, depending on the strain. The diameter of the clear zone shown on plates was accurately measured and it is the antimicrobial activity expressed in cm. Sterile deionised water was used as a negative control; the standard antibacterial agent, chloramphenicol, was used as a positive as in Dall'Agnol et al. « Antimicrobial activity of some Hypericum species » Phytomedicine 10: 5 1 1-5 16. (2003).

### RESULTS

### a) Antimicrobial activity against other Lactobacillus spp.

The clones did not exhibit antimicrobial activity against Lactobacillus sakei 20494 (except the clone 57 at a very low degree).
- Clones 37, 38 and 48 exhibited a low inhibitory activity against Lactobacillus casei ATCC 9595, L. bulgaricus ATCC 11842, L. fermentum DSM 20052, L. rhamnosus DSM 2071 1
- Clone 41 exhibited a low inhibitory activity against L. fermentum DSM 20052, L. rharnnosus DSM 2071 1, L. acidophilus DSM 20079
- Clone 43 exhibited a low inhibitory activity against L. fermentum DSM 20052 and L. acidophilus DSM 20079

This result can be considered usual, due to the production by all lactic acid bacteria of antimicrobial substances, like bacteriocins, which can act not only against pathogenic bacteria, but, as "territorial defence mechanism" also against other Lactobacilli.

### b) Antimicrobial activity against pathogen bacteria.

The results are shown in the following table:

| | 32 | 34 | 37 | 38 | 41 | 43 | 48 | 57 |
|---|---|---|---|---|---|---|---|---|
| B cereus GN101 | ND | ND | OX | OX | OX | ND | OX | ND |
| B cereus DSM 4313 | X | X | X | X | ND | ND | X | X |
| B cereus DSM 4384 | ND | ND | OX | OX | ND | OX | OX | ND |
| Ent. Faecalis ATCC 29212 | ND | ND | XX | XX | XX | XX | XX | ND |
| E coli HB101 | ND | X | X | X | ND | ND | X | ND |
| E coli DSM 8579 | ND | ND | X | X | ND | ND | X | OX |
| Ps aeruginosa DSM 50071 | ND | ND | XXX | X | XX | ND | X | ND |

| | | | | | | | | |
|---|---|---|---|---|---|---|---|---|
| legend: ND not detectable OX low inhibitory activity (halo: < 5mm) X medium inhibitory activity (halo: 6-8 mm) XX discrete inhibitory activity (halo from 8 to 10 mm) XXX strong inhibitory activity (halo > 10 mm) | | | | | | | | |

It is interesting to observe the strong antimicrobial activity exhibited by clone 37 against Ps. Aeruginosa. Clones 37, 38 and 48 exhibited a certain antimicrobial activity against the toxinogenic strain E coli DSM 8579.

### 6) Antibiotic sensitivity

The sensitivity of colonies to different antibiotics was evaluated by using 30 mg to 40 mg of the following antibiotics:
- ampicillin, and amoxicillin as inhibitor of cell wall synthesis;
- gentamicin sulfate, lincomycin, streptomycin sulfate, tetracycline, chloramphenicol and spiramycin as inhibitors of protein synthesis;
- rifamixin as inhibitors of nucleic acid synthesis.

Each of the antibiotic powders was carefully weighed, dissolved, diluted in appropriate diluents and filter sterilized prior to addition to MRS medium. Plates were inoculated with LAB strains, and incubated as above described. The sensitivity or resistance to antibiotics was evaluated by the inhibition halo test.

### RESULTS

The clones exhibited a variable degree of sensitivity to the antibiotics used in the test. As shown in the table below, clone 32 was sensitive to all antibiotics; on the other hand, the other clones were more or less resistant against streptomycin and lyncomycin.

| Clone | Antibiotic sensitivity |
|---|---|
| 32 | sensitivity to all antibiotics tested |
| 34 | sensitivity to all antibiotics tested |
| | resistance against streptomycin |
| | low resistance against lincomycin |
| 37 | sensitivity to all antibiotics tested |
| | low resistance against streptomycin and lincomycin |
| 38 | sensitivity to all antibiotics tested |
| | resistance against streptomycin and lincomycin |
| 41 | sensitivity to all antibiotics tested |
| | resistance against streptomycin |
| | low resistance against lincomycin |
| 43 | sensitivity to all antibiotics tested |
| | resistance against streptomycin |
| | low resistance against lincomycin |
| 48 | sensitivity to all antibiotics tested |
| | resistance against streptomycin |
| | low resistance against lincomycin |
| 57 | sensitivity to all antibiotics tested |
| | resistance against streptomycin |
| | low resistance against rifamixin |

### 7) Production of organic acids

The production of organic acids after fermentation was determined by high-performance liquid chromatography of filtered supernatants (Vulevic, Rastall & Gibson, 2004; Nazzaro et al. 2009), using a Gold System apparatus equipped with an ultraviolet detector (Beckman, CA, USA). Samples were loaded onto a pre-packed Aminex HPX-87 column (300 7.8 mm, Bio-Rad, Hercules, CA, USA) and eluted with 0.005 mM sulphuric acid. Total running time was 40 min; flow rate was 0.6 ml/min; injection volume was 20 µl; and detection wavelength was 210 nm.

### RESULTS

In the following tables are reported the amount of butyric acid and citric acid produced during fermentation by strains of the invention and some strains of reference, grown in donkey milk. The data are expressed in terms of absolute area (mm²):

| Strain | Amount of butyric acid |
|---|---|
| Lactobacillus acidophilus (comp.) | 26.21 |
| Lactobacillus plantorum (comp.) | 21.5 |
| Lactobacillus fermentum (comp.) | 33.56 |
| Clone 32 (inv.) | 60.3 |
| Clone 37 (inv.) | 65.41 |
| Clone 38 (inv.) | 42.91 |
| Clone 48 (inv.) | 60.37 |

| Strain | Amount of citric acid |
|---|---|
| Lactobacillus acidophilus (comp.) | 51.37 |
| Lactobacillus plantorum (comp.) | 2.95 |
| Lactobacillus fermentum (comp.) | 57.97 |
| Clone 32 (inv.) | 65.81 |
| Clone 37 (inv.) | 82.1 |
| Clone 38 (inv.) | 83.11 |
| Clone 41 (inv.) | 76.2 |
| Clone 43 (inv.) | 79.98 |
| Clone 48 (inv.) | 85.71 |

The production of butyric acid could be made externally by fermenting food in which bacteria of the invention have been inoculated, such food being then ingested by the user in order to increase the butyric acid in the digestive system.

Butyric acid is a short chain fatty acid that may also be produced from the fermentation by the bacteria in the colon, and meets 60% to 70% of the energetic needs of the epithelial cells of colon. Moreover, butyric acid stimulates regeneration of the epithelium of colon, jejunum, ileum, and inhibits the microbial growth in colon. A poor metabolism of butyric acid by the epithelium can cause ulcerative inflammation of colon.

The table shows that the strains of the invention are able to produce a higher amount of butyric acid than reference strains. Therefore, the strains of the invention allow to stimulate the intestinal barrier and to strengthen the immune system.

Citric acid is used as natural preservative in foods and drinks. The table shows that the strains of the invention are able to produce a higher amount of citric acid than reference strains. Therefore, they allow to improve the storage properties of composition comprising the strains of the invention.

### 8) Evolution of the pH during incubation

The microbial growth in donkey milk was tested. Donkey milk was obtained, in spray dried form, from the Eurolactis farm. Milk was re-suspended in sterile deionised water (rate 100 gr + 600 ml of water). 42 ml of such suspension were inoculated with 0.840 ml of a microbial culture of the isolated (2% inoculum, initial absorbance at λ ₆₀₀ₙₘ=1). Samples were incubated at 30°C.

Donkey milk was inoculated in the same conditions with Lactobacillus plantarum, Lactobacillus paraplantarum and Lactobacillus pentosus type-strains, while a temperature of 37°C was utilized for Lactobacillus bulgaricus and Lactobacillus rhamnosus.

The capability to grow into donkey milk and the fermentative ability of strains were evaluated by the Colony Forming Units (CFU)/ml counting of microbial cells (determined by anaerobic culturing on MRS agar after 3, 6 and 24 hours of incubation) and by measuring the resulting pH, respectively.

### RESULTS

The initial pH value was 7.14.

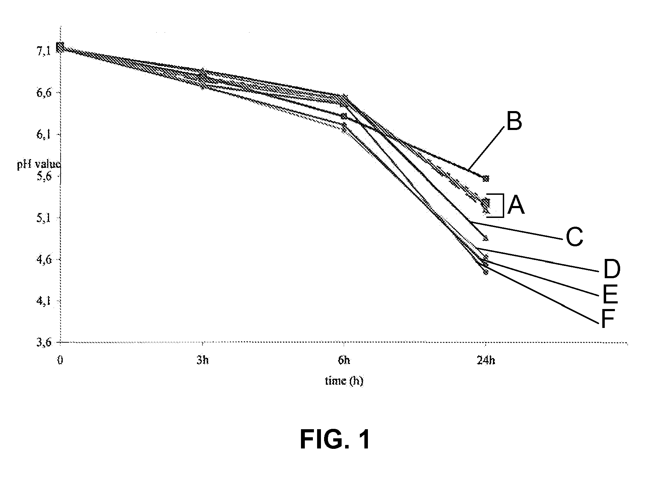

Figure 1 shows the decrease of pH during 24 hours of incubation of clones of the invention and reference strains in donkey milk. Reference A designates the group of the different strains of the invention. References B to F correspond respectively to L. bulgaricus, L. rhamnosus, L. paraplantarum, L. pentosus, and L. plantorum.

Figure 1 shows that the reference strains exhibited a more acidic-fermentative capability, compared to the clones of the invention. The palatability of the product is negatively affected by low pH. Therefore, the clones of the invention allow to preserve the palatability of the compositions comprising such clones.

Phenotypic and genotypic approaches (Metabolism of sugars, DNA fingerprint, DNA-DNA Hybridation, 16 S RNA sequence comparison)

### 9) Metabolism of sugars

Isolates were identified to the species level using the carbohydrate fermentation API 50 CHL (BioMérieux) identification system. API 50 CHL Medium, intended for the identification of the genus Lactobacillus and related organisms, is a ready-to use medium which enables the fermentation of 49 carbohydrates on the API 50 CH strip to be studied. A suspension is made in the medium with the microorganism to be tested and each tube of the strip is inoculated. During incubation, carbohydrates are fermented to acids which produce a decrease in the pH, detected by the colour change of the indicator. The results make up the biochemical profile of the strain and are used in its identification or typing. The presumed probiotic strains were inoculated in API 50 CHL strips and evaluation of colour changes was performed after 24 and 48 h of incubation at 37°C.

Each strip was composed as shown in the table below. The results were analysed by using an API BioMérieux software.

| **STRIP 0-9** | | **STRIP 10-19** | | **STRIP 20-29** | |
|---|---|---|---|---|---|
| **tube / substrate** | | **tube / substrate** | | **tube / substrate** | |
| 0 | CONTROL | 10 | GALactose | 20 | α-Methyl-D-Mannoside |
| 1 | GLYcerol | 11 | GLUcose | 21 | α-Methyl-D-Glucoside |
| 2 | ERYthrytol | 12 | FRUctose | 22 | N-Acetyl-Glucosamine |
| 3 | D ARAbinose | 13 | MaNnosE | 23 | AMYgdalin |
| 4 | L ARAbinose | 14 | SorBosE | 24 | ARButin |
| 5 | RIBose | 15 | RHAmnose | 25 | ESCulin |
| 6 | D XYLose | 16 | DULcitol | 26 | SALicin |
| 7 | L XYLose | 17 | INOsitol | 27 | CELlobiose |
| 8 | ADOnitol | 18 | MANnitol | 28 | MALtose |
| 9 | β-Methyl-D-Xyloside | 19 | SORbitol | 29 | LACtose |

| **STRIP 30-39** | | **STRIP 40-49** | | | |
|---|---|---|---|---|---|
| **tube / substrate** | | **tube / substrate** | | | |
| 30 | MELibiose | 40 | D TURanose | | |
| 31 | Sucrose | 41 | D LYXose | | |
| 32 | TREhalose | 42 | D TAGatose | | |
| 33 | INUlin | 43 | D FUCose | | |
| 34 | MeLeZitose | 44 | L FUCose | | |
| 35 | RAFfinose | 45 | D ARabitoL | | |
| 36 | Starch | 46 | L ARabitoL | | |
| 37 | GLYcoGen | 47 | GlucoNaTe | | |
| 38 | XyLiTol | 48 | 2-Keto-Gluconate | | |
| 39 | GENtobiose | 49 | 5-Keto-Gluconate | | |

| | | | | | |
|---|---|---|---|---|---|
| RESULTS of fermentation tests (API) Clone 32: insufficient discrimination Clone 34 : insufficient discrimination Clone 37: excellent identification with Lactobacillus plantarum 99.5% Clone 38: doubt identification Clone 41: preliminary doubt identification Clone 43: preliminary doubt identification Clone 48: excellent identification with Lactobacillus plantarum 99.5% Clone 57: insufficient discrimination | | | | | |

### Genetic approach

The following methodologies were applied for the molecular identification: DNA fingerprint, DNA-DNA Hybridization and 16 S RNA sequencing.

### 10) DNA fingerprinting

Random Amplified Polymorphic DNA-PCR (RAPD-PCR) assay was used to produce fingerprint patterns according to Ronimus et al. (1997). DNA amplification was performed in a 50 µL PCR reaction mixture containing: 50-200 ng of genomic DNA, 1X PCR buffer (supplied as component of the DNA polymerase kit), 3 mM MgCl₂, 250 µM dNTPs, 0.5 µM of OPR-2 primer (5'-CACAGCTGCC-3', sequence SEQ ID NO: 1) or OPR-13 primer (5'-GGACGACAAG- 3', sequence SEQ ID NO: 2) and 2.5 units of Platinum® Taq DNA polymerase (INVITROGEN). The mixtures were amplified in a thermocycler iCycler® (BIO RAD). The amplification profile consisted of an initial denaturation of 2 min at 92°C and 35 cycles of 15 sec at 94°C, annealing for 15 sec at 36°C (previously optimized by temperature gradient amplification) and elongation for 2 min at 72°C. A final extension of 7 min was carried out at 72°C. 10-20 µL of PCR products were electrophoresed on DNA 7500 microchip (Agilent) using a 2100 Bioanalyzer equipped by a 2100 EXPERT software (Agilent).

### RESULTS

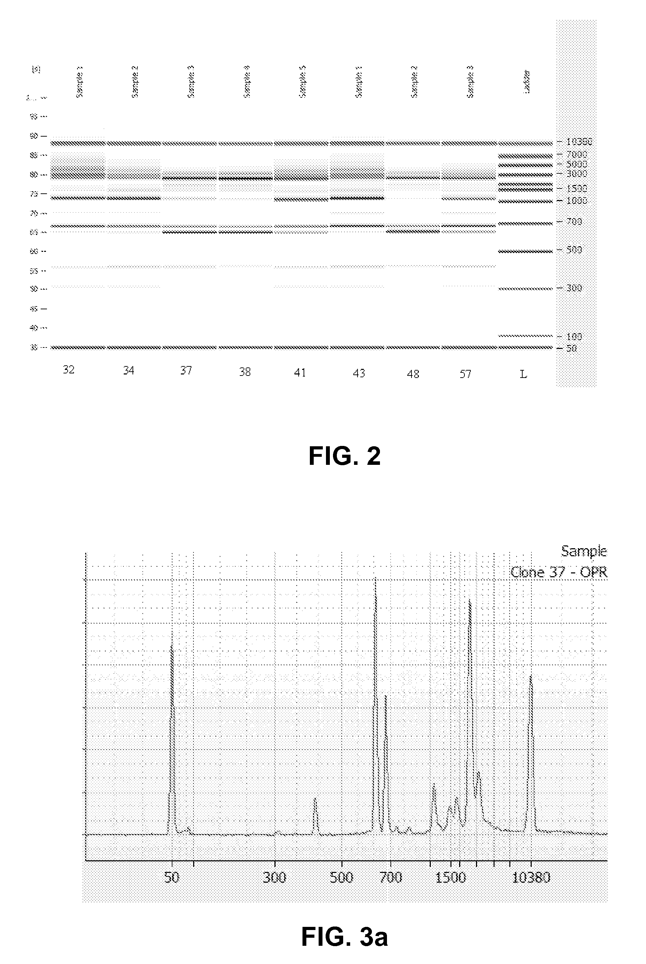

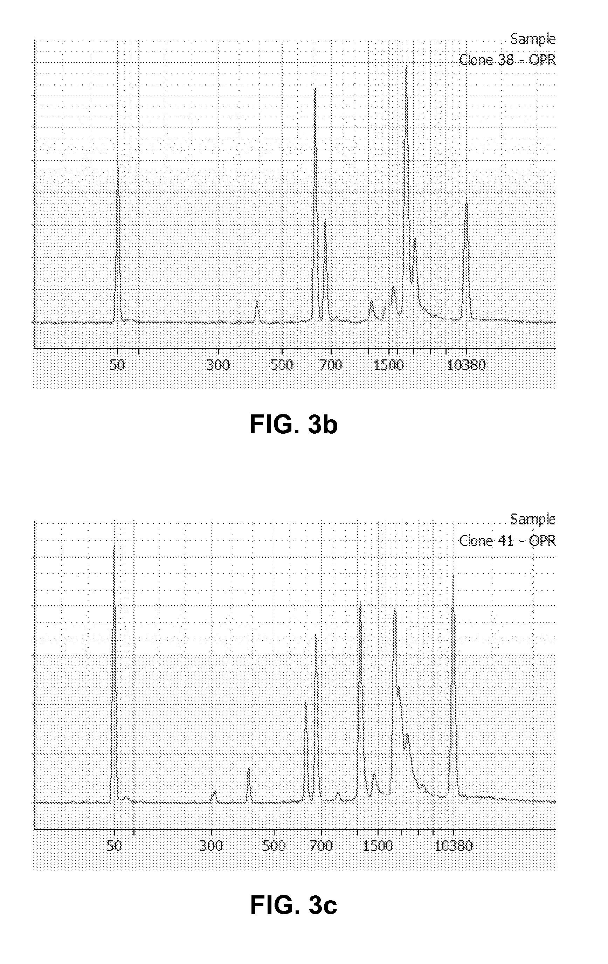

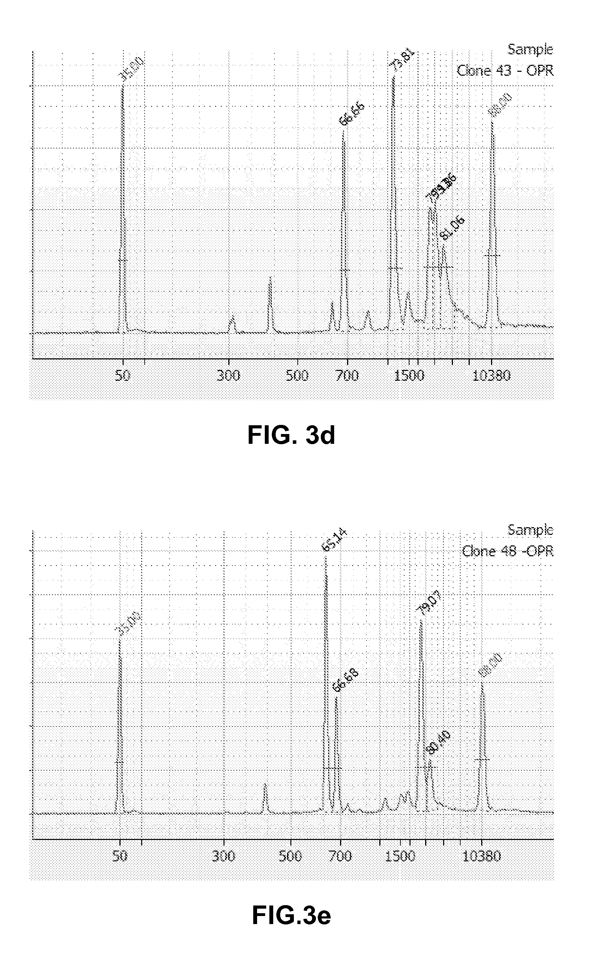

The API tests were followed by genotypic studies. DNA fingerprinting analysis (Figure 2) shows that clones 37, 38 and 48 seem to belong to the same species. In Figures 3a to 3e, the electropherograms relative to clones 37, 38, 41, 43 and 48 are provided.

### 11) 16 S RNA sequence comparison

Total RNA was extracted by RiboPure-Bacteria kit (Ambion) following manufacturer's instructions. RNA was dissolved in RNA-storage-solution (Ambion), UV-quantified by a Bio-Photometer® (Eppendorf) and stored to - 80°C. RNA aliquots (6 µg) were digested by RNAse-free DNAse I (Ambion DNA-freeTMk it) in a 20 µL final volume reaction mixture, to remove contaminating genomic DNA. After DNAse digestion, concentration and purity of RNA samples were evaluated by the RNA-6000-Nano® microchip assay, using a 2100 Bioanalyzer® equipped with a 2100-Expert-Software® (Agilent), following the manufacturer' s instructions. For all samples tested the RNA integrity number (R.I.N.) was greater than 7 (relatively to a 0-10 scale). were reverse transcribed in a 20 µL reaction mixture. A mixture containing 3 µg of total RNA, as evaluated by the 2100 Bioanalyzer, 2 pmoles of 1517R forward primer (see before), 2 mM dNTPs in a total volume of 10 µL was incubated for 2 min at 70°C and quickly cooled to 40°C. To the mixture were added 10 µL containing 2x of a suitable buffer (Invitrogen) 20 mM dithiothreitol, 20 units of RNAse inhibitor (Invitrogen), and 200 units of MoMuLV Superscript@ III reverse transcriptase (Invitrogen). The reaction mixture (final volume 20 µL) was incubated at 55°C for 60 min and at 70°C for 15 min to stop the reaction and for RNA degradation. Amplification of 16S cDNA was performed by an iCycler-iQ5® in a 50 µL reaction mixture containing: 1x of a suitable buffer (Invitrogen), 200 pM dNTPS, 300 nM of bacterial 16S general primers designed on E. coli 16S RNA sequence accession (8-Forward and 1517-Reverse). The amplification profile consisted of an initial denaturation of 3 min at 94°C, 25 cycles of 30 sec. at 94°C, annealing for 30 sec. 57°C and elongation for 1 min at 72°C and final elongation of 7 min at 72°C. 5 µL of PCR products were electrophoresed on 1% agarose gel (Agarose -1000, Invitrogen) in 1x TAE buffer at 5 V/cm for 4h. Ethidium bromide (0.1 µg/mL) was included both in the gel and electrophoresis buffer and PCR products were detected by UV visualization and recorded on Polapan 55 films (Polaroid). 100-150 µL of each amplification mixture were air dried and send to MWG (Ebersberg - Germany), for sequencing. Sequencing was performed using the primers 8F, 1517 R and by the internal primer 368 F, designed on the basis of the first sequencing data. Sequence-conting was performed by the DNA-Baser vers. 2.0 software utilizing the ".scf" sequence files. Generated ".fasta" sequences (about 1400 bases long) were aligned at NBCI -BLAST and at "Ribosome Data Base Project" -RDP data banks.

### RESULTS

Ribosomal RNA was extracted and retro-transcribed. cDNA obtained was amplified and sequenced as described above. Sequence-contings of about 1400 bases were obtained and compared in data banks. Below is a synthesis of sequence comparisons at Ribosomal Database Project (RDP). A picture is emerging that includes our clones into the very tight cluster of L. plantarum, paraplantarum and pentosus species that present (type strains) a 16 S RNA identity greater than 99.9%. The results given below are percentages of 16 S RNA identity of the clones of the invention compared to these species.
Clone 41
   domain Bacteria (20) (match sequences)
      phylum Firmicutes (20) class "Bacilli" (20) order "Lactobacillales" (20) family Lactobacillaceae (20) genus Lactobacillus (20)
      0.994 1400 L. paraplantarum (T); DSM 10667T; A5306297
      0.979 1405 L. plantarum (T); JCM 1 149; D792 10
      0.984 1410 L. pentosus (T); JCM 1558; D79211
Clone 43
   domain Bacteria (20) (match sequences)
      phylum Finnicutes (20) class "Bacilli" (20) order "Lactobacillales" (20) family Lactobacillaceae (20) genus Lactobacillus (20)
      1400 L. paraplantarum (T); DSM 10667T; AJ306297
      0.985 1405 L. plantarum (T); JCM 1149; D79210
      0.990 1410 L. pentosus (T); JCM 1558; D79211
Clone 48
   domain Bacteria (20) (match sequences)
      phylum Firmicutes (20) class "Bacilli" (20) order "Lactobacillales" (20) family Lactobacillaceae (20) genus Lactobacillus (20)
      1.000 L. plantarum (T); JCM 1 149; D792 10
Clone 57
   domain Bacteria (0/20/5164) (selected/match/total RDP sequences)
      phylum Firmicutes (0/20/1178) class "Bacilli" (0/20/697) order "Lactobacillales" (0/20/289) family Lactobacillaceae (0/20/114) genus Lactobacillus (0/20/104)
      0.987 1400 L. paraplantarum (T); DSM 10667T; A5306297
      0.990 1405 L. plantarum (T); JCM 1149; D79210
      0.994 1410 L. pentosus (T); JCM 1558; D79211

### 12) DNA-DNA Hybridization

For Quantitative DNA-DNA hybridisation and homology percentage calculation the DNA was extracted and purified from bacterial cell culture (about 250 mg of dry pellet for each strain) using the Genomic-DNA-Buffer Set and the Genomic-tip-100/G columns (QIAGEN SPA, Milano Italy), according to manufacturer's instructions with minor modifications. DNA was dissolved in TE buffer (10 mM Tris pH 8, 1 mm EDTA) and serial diluted to a final concentration (WS) of 50 µg/mL, as evaluated by UV-absorbance using a Bio-Photometer (Eppendorf, Germany). WS DNA concentration was confirmed by fluorimetric measurements using the Quant-iT DNA assay Kit (INVTTROGEN, Milano Italy); DNA size was estimated by 0.8% DNA-grade agarose (BIO-RAD) electrophoresis using DNA as molecular weight marker (DNAs size about 32 kD). WS solutions were diluted to a final concentration of 2 ng/mL in 0.1 X SSC containing 5 ng/mL herring sperm DNA. DNA was denatured by 10 min at 100°C and quick immersion in water-ice bath. 50-100 ng of DNA for each strain were blotted in quadruplicate on nylon membrane positively charged (Roche, Germany) by using a Dot-Blot apparatus (Bio-Rad, Ca USA) connected to a soft vacuum. A standard curve 20 to 200 ng DNA/spot of homologous DNA (the DNA to probe) was included in the dot. The DNA was cross-linked to nylon by 3 min UV exposure and by 1 hr backing under-vacuum at 120°C. Membranes were frozen at -20°C until analysis.

1 µg of DNA-to-probe was labelled overnight with digoxigenin-dUTP in a 20 µL reaction mixture using the Random Primed DNA labelling kit (Roche, Germany) according to manufacturer's instructions. Membranes were pre-hybridizated for 3 hrs at 40°C in DIG Easy-Hyb solution (Roche) and hybridizated over-night at 40°C in DIG Easy-Hyb solution containing 20 pg/mL of Dig-labelled probe (heat-denatured as described above), using a tube-rotating hybridization incubator (GFL). Stringency washes were : twice for 5 min at room temperature in 2 x SSC solution containing 0.1 SDS, twice for 15 min at 68°C in 0.1 x SSC solution containing 0.1 x SDS. Immune-detection was performed using the anti-Digoxigenin-AP antibody (anti-digoxigenin FAB fragment conjugated to alkaline-phosphatase), the CDP-Star chemiluminescent substrate and the DIG Wash and Block buffer set Kit, all reagents and relative instructions were from Roche. Chemiluminescence was quantified in condition of time-exposure linearity by using a VersaDOC 4000 apparatus (BIO-RAD) equipped by the Quantity-one software. The DNA-DNA homology percentage was calculated according to Jahnke (1994) by putting as 100 % the media of the chemiluminescence values (Adjusted Volume Intensity x mm²) acquired from the homologous DNA spots and taking in account the linear response of the homologous DNA standard curve. The media standard deviation of replicate samples did not exceeded 5% of the media value.

### RESULTS

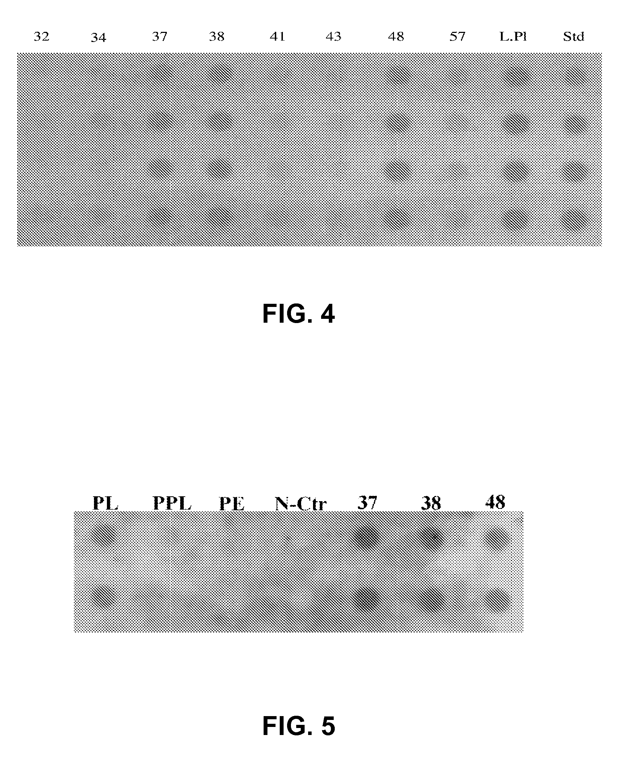

Metabolic data were confirmed by DNA-DNA hybridization, utilizing L. plantarum DNA labelled by dUTP-dioxigenin as a probe. As shown by Figure 4, the clones 37, 38, 48 showed 86, 99, 99 % respectively of DNA-DNA homology with L. plantarum (L. PI on Figure 4), knowing that the taxonomic DNA-DNA limit for different species is below 70% of DNA-DNA homology.

To distinguish from the three possibilities and to confirm previous findings, clones 37, 38 and 48 were DNA-DNA hybridised within L. plantarum (PL on figure 5), L. paraplantarum (PPL on figure 5) and L. pentosus (PE on figure 5) strain-type DNAs, to a probe of labelled DNA from clone 37 (Fig 5). N-Ctr is negative control.

The foregoing description of preferred embodiments of the invention is not intended to be exhaustive or to limit the invention to the disclosed embodiments. Various changes within the scope of the invention will become apparent to those skilled in the art and may be acquired from practice of the invention.

Based on the experimental results of DNA-DNA hybridization and the analysis of 16S RNA, the strains 37, 38 and 48 belong to a unique cluster, in which L. plantarum, L. pentosus and L. paraplantarum are also present, but are different from them. It is considered that strains 37, 38 and 48 represent a subspecies of L. plantarum, which will be named L. plantarum asini. For similar reasons, the strains 41 and 43 apparently belong to a new species, Lactobacillus asini. Indeed, strains 41 and 43 present a 40-50% of similarity with the other lactobacilli, mainly with pentosus, plantarum and paraplantarum.

Strains 37, 38 and 48, and strains 41 and 43, are microorganisms belonging to genus Lactobacillus, with a homology of the 16S RNA higher than 99.2% with the strict cluster of L plantarum , L paraplantarum and L pentosus, and with relative finger print and protein profile proportional to such homology.

### SEQUENCE LISTING

SEQ ID NO: 1
   LENGTH: 10 bases
   TYPE: nucleic acid
   STRANDENESS: single
   TOPOLOGY: linear
   MOLECULAR TYPE: DNA
   SEQUENCE:
   CACAGCTGCC
SEQ ID NO: 2
   LENGTH: 10 bases
   TYPE: nucleic acid
   STRANDENESS: single
   TOPOLOGY: linear
   MOLECULAR TYPE : DNA
   SEQUENCE:
   GGACGACAAG

## Claims

1. Probiotic lactic bacteria, isolated particularly from Donkey milk, and selected from the group consisting of strains deposited under accession number DSM 22098, DSM 22099, DSM 22100, DSM 22102, DSM 22101, DSM 23558, DSM 23559, and DSM 23560 with DSMZ.

2. Composition comprising one or a plurality of the probiotic lactic bacteria species or strains according to claim 1.

3. Composition according to claim 2, wherein the composition is a food composition or a beverage composition.

4. Process for manufacturing a food composition or a beverage composition containing at least the steps of:
- inoculating a food product with viable probiotic lactic bacteria according to claim 1
- placing the inoculated food product under conditions favorable to the metabolism of said probiotic lactic bacteria
- fermenting said food product until a population of at least [10⁶] CFU / mL of food product is reached.

5. Process for manufacturing a food composition or a beverage composition containing at least the steps of:
- fermenting probiotic lactic bacteria according to claim 1 until a population of at least [10⁶] CFU / mL is reached,
- protecting said probiotic lactic bacteria,
- admixing a food product with said protected probiotic lactic bacteria.

6. A process according to claim 4 or 5, wherein fermentation is stopped when it has reached the stationary phase of fermentation, preferably within about 24 hours of having placed said probiotic lactic bacteria under conditions favorable to their metabolism.

7. Use of probiotic lactic bacteria according to claim 1 in the preparation of a composition for treating a disorder associated with the colonization of a mucous membrane by a pathogenic microorganism.

8. Use according to claim 7, wherein said mucous membrane is selected in the group consisting of the gut mucosa and the stomach mucosa.

9. Use according to claim 7 or 8, wherein said pathogenic microorganisms is an enteropathogens, such as *Salmonella enteriditis, Vibrio cholerae, Escherichia coli.*

10. Use of probiotic lactic bacteria according to claim 1 for protecting fermented food products against food pathogens, such as *Lysteria* or *Salmonella, Campylobacter* or *Clostridium,* by inhibiting the development of such food pathogens.

11. Use of probiotic lactic bacteria according to claim 1 for producing butyric acid during fermentation making use of said bacteria.

12. Method for producing butyric acid comprising the steps of fermenting probiotic lactic bacteria according to claim 1 under suitable conditions and collecting the butyric acid produced during fermentation by said bacteria.

## Patentansprüche

1. Probiotische Milchbakterien, die insbesondere aus Eselsmilch isoliert sind und aus der Gruppe ausgewählt sind, bestehend aus den Stämmen, die beim DSMZ unter der Hinterlegungs-Nr. DSM 22098, DSM 22099, DSM 22100, DSM 22102, DSM 22101, DSM 23558, DSM 23559 und DSM 23560 hinterlegt sind.

2. Zusammensetzung, die eine/n oder eine Mehrzahl der probiotischen Milchbakterienspezies oder -stämme nach Anspruch 1 umfasst.

3. Zusammensetzung nach Anspruch 2, wobei die Zusammensetzung eine Lebensmittelzusammensetzung oder eine Getränkezusammensetzung ist.

4. Prozess zum Herstellen einer Lebensmittelzusammensetzung oder einer Getränkezusammensetzung, der zumindest die Schritte beinhaltet:
- Beimpfen eines Lebensmittelprodukts mit lebensfähigen probiotischen Milchbakterien nach Anspruch 1,
- Setzen des beimpften Lebensmittelprodukts unter Bedingungen, die den Stoffwechsel der probiotischen Milchbakterien begünstigen,
- Fermentieren des Lebensmittelprodukts, bis eine Population von zumindest [10⁶] KbE/ml Lebensmittelprodukt erreicht ist.

5. Prozess zum Herstellen einer Lebensmittelzusammensetzung oder einer Getränkezusammensetzung, der zumindest die Schritte beinhaltet:
- Fermentieren von probiotischen Milchbakterien nach Anspruch 1, bis eine Population von zumindest [10⁶] KbE/ml erreicht ist,
- Schützen der probiotischen Milchbakterien,
- Vermischen eines Lebensmittelprodukts mit den geschützten probiotischen Milchbakterien.

6. Prozess nach Anspruch 4 oder 5, wobei die Fermentation gestoppt wird, wenn die stationäre Phase der Fermentation erzielt wurde, vorzugsweise innerhalb von 24 Stunden nach Setzen der probiotischen Milchbakterien unter für deren Stoffwechsel günstige Bedingungen.

7. Verwendung von probiotischen Milchbakterien nach Anspruch 1 bei der Herstellung einer Zusammensetzung zum Behandeln einer Störung, die mit der Kolonisierung einer Schleimhaut durch einen pathogenen Mikroorganismus assoziiert ist.

8. Verwendung nach Anspruch 7, wobei die Schleimhaut aus der Gruppe ausgewählt ist, bestehend aus Darmschleimhaut und Magenschleimhaut.

9. Verwendung nach Anspruch 7 oder 8, wobei die pathogenen Mikroorganismen Enteropathogene sind, z. B. Salmonella enteriditis, Vibrio cholerae, Escherichia coli.

10. Verwendung von probiotischen Milchbakterien nach Anspruch 1 zum Schützen von fermentierten Lebensmittelprodukten vor Lebensmittelpathogenen, z. B. Lysteria oder Salmonella, Campylobacter oder Clostridium, durch Hemmen der Entwicklung solcher Lebensmittelpathogene.

11. Verwendung von probiotischen Milchbakterien nach Anspruch 1 zum Herstellen von Buttersäure während der Fermentation unter Verwendung der Bakterien.

12. Verfahren zum Herstellen von Buttersäure, das die Schritte des Fermentierens von probiotischen Milchbakterien nach Anspruch 1 unter geeigneten Bedingungen und des Sammelns von Buttersäure umfasst, die während der Fermentation durch die Bakterien erzeugt wurde.

## Revendications

1. Bactéries lactiques probiotiques isolées particulièrement à partir de lait d'ânesse et sélectionnées dans le groupe consistant en les souches déposées sous le numéro d'accès DSM 22098, DSM 22099, DSM 22100, DSM 22102, DSM 22101, DSM 23558, DSM 23559 et DSM 23560 auprès de la DSMZ.

2. Composition comprenant une seule ou une pluralité des espèces ou souches de bactéries lactiques probiotiques selon la revendication 1.

3. Composition selon la revendication 2, où la composition est une composition alimentaire ou une composition de boisson.

4. Procédé de fabrication d'une composition alimentaire ou d'une composition de boisson contenant au moins les étapes qui consistent à :
- inoculer un produit alimentaire avec des bactéries lactiques probiotiques viables selon la revendication 1
- placer le produit alimentaire inoculé dans des conditions favorables au métabolisme desdites bactéries lactiques probiotiques
- fermenter ledit produit alimentaire jusqu'à atteindre une population d'au moins [10⁶] UFC/ml de produit alimentaire.

5. Procédé de fabrication d'une composition alimentaire ou d'une composition de boisson contenant au moins les étapes qui consistent à :
- fermenter des bactéries lactiques probiotiques selon la revendication 1 jusqu'à atteindre une population d'au moins [10⁶] UFC/ml,
- protéger lesdites bactéries lactiques probiotiques,
- mélanger un produit alimentaire avec lesdites bactéries lactiques probiotiques protégées.

6. Procédé selon la revendication 4 ou 5, dans lequel la fermentation est interrompue lorsque la phase stationnaire de fermentation est atteinte, de préférence en moins d'environ 24 heures après avoir placé lesdites bactéries lactiques probiotiques dans des conditions favorables à leur métabolisme.

7. Utilisation de bactéries lactiques probiotiques selon la revendication 1 dans la préparation d'une composition destinée au traitement d'un trouble associé à la colonisation d'une muqueuse par un micro-organisme pathogène.

8. Utilisation selon la revendication 7, dans laquelle ladite muqueuse est sélectionnée dans le groupe consistant en la muqueuse intestinale et la muqueuse gastrique.

9. Utilisation selon la revendication 7 ou 8, dans laquelle ledit micro-organisme pathogène est un entéropathogène, comme Salmonella enteriditis, Vibrio cholerae, Escherichia coli.

10. Utilisation de bactéries lactiques probiotiques selon la revendication 1 pour protéger des produits alimentaires fermentés contre des agents pathogènes alimentaires, comme Listeria ou Salmonella, Campylobacter ou Clostridium, en inhibant le développement de tels agents pathogènes alimentaires.

11. Utilisation de bactéries lactiques probiotiques selon la revendication 1 pour produire de l'acide butyrique pendant une fermentation utilisant lesdites bactéries.

12. Procédé de production d'acide butyrique, comprenant les étapes de fermentation de bactéries lactiques probiotiques selon la revendication 1 dans des conditions appropriées et de collecte de l'acide butyrique produit pendant la fermentation par lesdites bactéries.
